# EUROPÄISCHE PATENTANMELDUNG

(11) **EP 4 643 925 A1**
(43) Veröffentlichungstag der Anmeldung: **05.11.2025**
(21) Anmeldenummer: 24173607.3
(22) Anmeldetag: 30.04.2024
(51) Int. Cl.: A61M 60/178, A61M 60/216, A61M 60/508, A61M 60/538, A61M 60/585, A61M 60/871, H02J 7/04

(54) **STEUEREINRICHTUNG FÜR EINE BLUTPUMPE**

(71) Anmelder: Berlin Heart GmbH, 12247 Berlin (DE)
(72) Erfinder: Frischke, Michael, 12247 Berlin (DE)
(74) Vertreter: Pfenning, Meinig & Partner mbB

(57) **Zusammenfassung**

Diese Offenbarung betrifft eine Steuereinrichtung (100) für eine Blutpumpe (190), die einen Blutpumpenanschluss (110) zur Herstellung einer elektrischen Verbindung zwischen der Steuereinrichtung (100) und der Blutpumpe (190) umfasst. Zudem umfasst die Steuereinrichtung (100) eine Energiespeichereinheit (120) zum Bereitstellen elektrischer Energie sowie eine Energiebereitstellungseinheit (130), die ausgebildet ist, für einen Betrieb der Blutpumpe (190) von der Energiespeichereinheit (120) bereitgestellte elektrische Energie am Blutpumpenanschluss (110) zur Verfügung zu stellen. Ferner umfasst die Steuereinrichtung (300) eine Ladeschnittstelle (160), die ausgebildet ist, elektrische Energie einer externen elektrischen Energiequelle zu empfangen, und eine Lademanagementeinheit (140), die ausgebildet ist, einen Füllstand (124) der Energiespeichereinheit (120) zu empfangen und zum Aufladen der Energiespeichereinheit (120) über die Ladeschnittstelle (160) empfangene elektrische Ladeleistung an der Energiespeichereinheit (120) bereitzustellen. Zudem weist die Steuereinrichtung (100) mindestens eine Betriebsparametererfassungseinheit (170) auf, die ausgebildet ist, einen Betriebsparameter (172) der Steuereinrichtung (100) zu ermitteln. Weiterhin ist die Lademanagementeinheit (140) ausgebildet ist, einen Wert (142) einer Ladespannung, einen Wert (142) eines Ladestrom, eine Ladeobergrenze (LW1, LW2) und/oder einen Alarmsignalschwellwert (AW1, AW2) unter Benutzung von mindestens einem der Betriebsparameter (172) festzulegen.

## Beschreibung

Diese Offenbarung betrifft eine Steuereinrichtung für eine Blutpumpe, eine Blutpumpeneinrichtung, die eine Blutpumpe und eine entsprechende Steuereinrichtung umfasst sowie ein Verfahren zum Betreiben einer Steuereinrichtung für eine Blutpumpe.

Um Patienten mit elektrisch betriebenen, medizinischen Geräten, wie bspw.

Herzunterstützungssystemen (engl. Ventricular Assist Device, kurz VAD), Mobilität zu ermöglichen, werden diese oftmals mit Energiespeichereinheiten ausgestattet. Aufgrund der Möglichkeit hohe Energiedichten zu realisieren, umfassen diese Energiespeichereinheiten oftmals Li-Ionen Akkumulatoren (kurz Akkus). Neben dem Vorteil hoher Energiedichten haben Li-Ionen Akkus auch eine Reihe von Nachteilen. So wurde festgestellt, dass die Lebensdauer von Li-Ionen Akkus besonders stark abnimmt, wenn die Akkus häufig vollständig geladen und vollständig entladen werden. Dies soll anhand des folgenden Beispiels illustriert werden.

Wird eine Zelle eines Lithium-Ionen-Akkus immer auf 100 % geladen (4.2 V Ladeschlussspannung), hält sie ca. 1000 Ladezyklen bis eine Gesamtkapazität der Akkuzelle auf etwa 80% gesunken ist. Wird die gleiche Akkuzelle jedoch immer nur auf 95% (ca. 4.1 V Ladeschlussspannung), hält sie ca. 1600 Ladezyklen bis die Gesamtkapazität auf 80% gesunken ist.

Aber nicht nur die Lebensdauer von Lithium-Ionen Akkus-sind problematisch, sondern auch deren thermisches Verhalten während des Aufladens. So kann es bei Defekten des Lithium-Ionen-Akkus dazu kommen, dass der Akku beim Aufladen überhitzt. Auch kann es durch ein Laden von Lithium-Ionen-Akkus bei hohen Temperaturen zu Defekten des Akkus kommen. Zudem kann es bei hohen Umgebungstemperaturen auch beim Aufladen eines Lithium-Ionen-Ackus, der nicht von einem Defekt betroffen ist, zu starken Verlustleistungen und damit zu einer Wärmeentwicklung kommen. Dies stellt ein Sicherheitsproblem nicht nur für den Benutzer des Gerätes sondern auch für dessen unmittelbare Umgebung dar.

Die Aufgabe, die der vorliegenden Offenbarung zugrunde liegt, ist einen Ansatz aufzuzeigen, die zumindest teilweise den oben beschriebenen Herausforderungen entgegenwirkt.

Dieser Ansatz wird verwirklicht durch die Steuereinrichtung gemäß Anspruch 1 und das Verfahren zum Betreiben einer Steuereinrichtung gemäß Anspruch 15.

Diese Steuereinrichtung umfasst einen Blutpumpenanschluss zur Herstellung einer elektrischen Verbindung zwischen der Steuereinrichtung und der Blutpumpe. Weiterhin umfasst die Steuereinrichtung eine Energiespeichereinheit zum Bereitstellen elektrischer Energie und eine Energiebereitstellungseinheit, die ausgebildet ist, für einen Betrieb der Blutpumpe von der Energiespeichereinheit bereitgestellte elektrische Energie am Blutpumpenanschluss zur Verfügung zu stellen. Zudem umfasst die Steuereinrichtung eine Ladeschnittstelle, die ausgebildet ist, elektrische Energie einer externen elektrischen Energiequelle zu empfangen, und eine Lademanagementeinheit, die ausgebildet ist, einen Füllstand der Energiespeichereinheit zu empfangen und zum Aufladen der Energiespeichereinheit über die Ladeschnittstelle empfangene elektrische Ladeleistung an der Energiespeichereinheit bereitzustellen. Weiterhin ist die Lademanagementeinheit ausgebildet, mindestens eine der drei folgenden Funktionen zu verwirklichen:
- einen Wert einer Ladespannung und/oder einen Wert eines Ladestrom für die an der Energiespeichereinheit bereitgestellte Ladeleistung unter Benutzung des Füllstands und mindestens einem der Betriebsparameter festzulegen, und/oder
- eine Ladeobergrenze, bis zu der die Energiespeichereinheit geladen wird, unter Benutzung von mindestens einem der Betriebsparameter festzulegen, und/oder
- einen Alarmsignalschwellwert, in Abhängigkeit dessen ein Alarmsignal bereitgestellt wird, unter Benutzung von mindestens einem der Betriebsparameter festzulegen.

Die Erfinder haben erkannt, dass gerade wenn die Energiespeichereinheit in der Steuereinrichtung fest verbaut ist, die Lebensdauer der Steuereinrichtung maßgeblich vom Verschleiß der Energiespeichereinheit abhängt. Wie oben beschrieben, kann es jedoch durch häufiges Be- und Entladen der Energiespeichereinheit besonders bei Lithium-Ionen-Akkus, zu einer Reduzierung der Ladekapazität kommen. Fällt eine Ladekapazität unter einen bestimmten Wert, kann dies erhebliche Einschränkungen bei der Benutzung der Vorrichtung mit sich bringen. Im Resultat kann dies zu einer kürzeren Gesamtlebenszeit der Vorrichtung führen. Weiterhin kann es beim Laden solch einer Energiespeichereinheit auch zu exothermen Reaktionen kommen, die bei fest verbauten Energiespeichereinheiten ein großes Sicherheitsrisiko für den Benutzer darstellen können. Diesen negativen Auswirkungen soll mit der oben beschriebenen Vorrichtung entgegengewirkt werden.

Durch das Einstellen der Ladespannung- und/oder des Ladestroms kann ein Laden der Energiespeichereinheit kurzfristig beendet oder zumindest reduziert werden, sollten anhand von dem einen oder den mehreren erfassten Betriebsparametern kritische Situationen detektiert werden.

Weiterhin kann durch das Festlegen von Ladeobergrenze und/oder Alarmschwellwert anhand von erfassten Betriebsparametern ein Laden und Entladen der Energiespeichereinheit so zu beeinflussen, dass eine Lebensdauer der Energiespeichereinheit möglichst lang ist und dennoch ein Einfluss auf den Benutzer der Vorrichtung minimal ist.

Im Folgenden werden weitere mögliche Ausführungsformen der Steuereinrichtung beschrieben.

In einer Ausführungsform kann die Energiespeichereinheit eine interne Energiespeichereinheit sein. In diesem Fall umfasst die Steuereinrichtung ein Steuereinrichtungsgehäuse, das die Steuereinrichtung umschließt. Durch einen Festeinbau der Energiespeichereinheit kann die Energiespeichereinheit platzsparender verbaut werden.

In einer anderen Ausführungsform der Steuereinrichtung kann die mindestens eine Betriebsparametererfassungseinheit zusätzlich oder alternativ zu den genannten optionalen Merkmalen eine Temperatursensoreinheit umfassen, die ausgebildet ist, als Betriebsparameter eine Umgebungstemperatur am Ort der Temperatursensoreinheit zu ermitteln. In dieser Ausführungsform kann zusätzlich die Lademanagementeinheit ausgebildet sein, den Wert der Ladespannung und/oder den Wert des Ladestroms in Abhängigkeit von der Umgebungstemperatur festzulegen. Diese Ausführungsform kann vorteilhaft sein, um zu verhindern, dass die Steuereinrichtung während des Ladens der Energiespeichereinheit überhitzt. Damit kann die Benutzersicherheit der Steuereinrichtung erhöht werden.

In einer Variante dieser Ausführungsform kann ferner die Abhängigkeit zwischen dem Wert der Ladeleistung und der Umgebungstemperatur so gewählt sein, dass der Wert der Ladeleistung umso geringer ist, je größer die Umgebungstemperatur ist. Dieses Merkmal kann dazu beitragen, ein Überschreiten einer kritischen Temperatur an oder innerhalb der Steuereinrichtung zu verhindern.

In einer anderen Variante kann zusätzlich oder alternativ zu dem zuvor beschriebenen Merkmal die Temperatursensoreinheit in einem Inneren des Steuergerätes angeordnet sein, in unmittelbarer Nähe zu einer Gehäuseoberfläche der Steuereinrichtung und/oder in einer unmittelbaren Nähe zu der elektrischen Energiespeichereinheit. Diese Anordnungen der Temperatursensoreinheit können hilfreich sein, die Temperatur der Steuereinrichtung an kritischen Punkten in und um die Steuereinrichtung zu überwachen.

In einer anderen Ausführungsform der Steuereinrichtung kann die mindestens eine Betriebsparametererfassungseinheit zusätzlich oder alternativ zu den optionalen Merkmalen der zuvor beschriebenen Ausführungsformen eine Ladequellenerkennungseinheit umfassen, die ausgebildet ist, als einen Betriebsparameter einen Ladequellentyp einer über die Ladeschnittstelle energiebereitstellenden externen elektrischen Energiequelle zu ermitteln. Ferner kann in dieser Ausführungsform die Lademanagementeinheit ausgebildet sein, den Wert der Ladespannung und/oder den Wert des Ladestroms in Abhängigkeit von dem Ladequellentyp festzulegen. So kann die Ladespannung und/oder der Ladestrom auf die externe Energiequelle abgestimmt werden.

In einer Variante dieser Ausführungsform kann die Ladequellenerkennungseinheit ausgebildet sein, als Ladequellentyp eine Netzversorgung und eine Speicherversorgung zu unterscheiden. Ferner kann die Abhängigkeit zwischen dem Wert des Ladestroms und/oder der Ladespannung und dem Ladequellentyp so gewählt sein, dass der Wert der Ladeleistung bei einer Netzversorgung höher ist als bei einer Speicherversorgung.

In einer anderen Ausführungsform kann die mindestens eine Betriebsparametererfassungseinheit zusätzlich oder alternativ zu den Merkmalen der zuvor besprochenen, optionalen Ausführungsformen ausgebildet sein, Leistungsabgabedaten in Abhängigkeit von einer über den Blutpumpenanschluss abgegebenen Leistung als einen Betriebsparameter bereitzustellen. Ferner kann bei dieser Ausführungsform die Lademanagementeinheit ausgebildet sein, die Ladeobergrenze und/oder den Alarmsignalschwellwert in Abhängigkeit von den Leistungsabgabedaten zu ermitteln. Dies kann vorteilhaft sein, um anhand einer Begrenzung der Ladung der Energiespeichereinheit und/oder der Entladung der Energiespeichereinheit einen Füllstand der Energiespeichereinheit innerhalb eines Fensters zu halten, in dem eine Maximalkapazität der Energiespeichereinheit nur wenig reduziert wird. Durch eine Anpassung des Fensters an die Leistungsabgabedaten kann gleichzeitig die Einschränkung der verwendbaren Speicherkapazität der Energiespeichereinheit so angepasst sein, dass der Einfluss auf den Benutzer minimiert wird. Der Füllstand der Energiespeichereinheit gibt dabei eine Menge der restlichen, in der Energiespeichereinheit gespeicherten Energie an.

In einer Variante dieser Ausführungsform kann die Abhängigkeit zwischen der Ladeobergrenze und den Leistungsabgabedaten so gewählt sein, dass die Ladeobergrenze niedriger ist, je geringer die von der Energiespeichereinheit abgegebene Leistung ist. Zusätzlich oder alternativ hierzu kann ferner die Abhängigkeit zwischen dem Alarmsignalschwellwert und den Leistungsabgabedaten so gewählt sein, dass der Alarmsignalschwellwert höher ist, je geringer die von der Energiespeichereinheit abgegebene Leistung ist. Diese Wahl der Abhängigkeit kann vorteilhaft sein, um die Energiespeichereinheit zu schonen und gleichzeitig einen Einfluss auf eine Möglichkeit die Steuereinrichtung zu benutzen so gering wie möglich zu halten.

In einer anderen Variante dieser Ausführungsform kann zusätzlich oder alternativ zu den zuvor genannten Merkmalen die die Leistungsabgabedaten bereitstellende Betriebsparametererfassungseinheit ausgebildet sein, die Leistungsabgabedaten durch Messung einer elektrischen Leistung, zu ermitteln. Hierbei kann die Messung beispielsweise am Blutpumpenabschluss, an der Energiespeichereinheit und/oder an der Ladeschnittstelle erfolgen.

In einer weiteren Variante kann zusätzlich oder alternativ die die Leistungsabgabedaten bereitstellende Betriebsparametererfassungseinheit ausgebildet sein, die Leistungsabgabedaten in Abhängigkeit davon bereitzustellen, ob eine Blutpumpe am Blutpumpenanschluss angeschlossen ist. Diese Art der Bestimmung der Leistungsabgabe kann besonders leicht realisierbar sein.

In einer weiteren Variante dieser Ausführungsform kann zusätzlich oder alternativ zu den Merkmalen der zuvor beschriebenen Varianten, die Lademanagementeinheit ausgebildet sein, den Alarmsignalschwellwert und/oder die Ladeobergrenze in Abhängigkeit einer Maximalkapazität der Energiespeichereinheit und der Leistungsabgabedaten festzulegen.

In dem oben beschriebenen Fall handelt es sich bei dem erhöhten Alarmsignalschwellwert um einen vorgelagerten Alarm, um eine akkuschonende Betriebsweise der Steuereinrichtung zu ermöglichen, handeln. Zusätzlich oder alternativ hierzu kann die Lademanagementeinheit auch ausgebildet sein, den Alarmsignalschwellwert unter Benutzung der Leistungsabgabedaten und einer gegebenen Mindestrestbetriebsdauer der Steuereinrichtung festzulegen. Der so bestimmte Alarmsignalschwellwert würde dann einem Alarmsignalschwellwert für einen Notalarm entsprechen.

In einer anderen Ausführungsform kann zusätzlich oder alternativ zu den Merkmalen der anderen, optionalen Ausführungsformen die mindestens eine Betriebsparametererfassungseinheit eine Energiespeicherzustandsermittlungseinheit umfassen, die ausgebildet ist, Energiespeicherzustandsdaten in Abhängigkeit eines Zustands der Energiespeichereinheit als einen Betriebsparameter bereitzustellen. Ferner kann bei dieser Ausführungsform die Lademanagementeinheit ausgebildet sein, die Ladeobergrenze und/oder den Alarmsignalschwellwert und/oder die Ladespannung und/oder den Ladestrom in Abhängigkeit von den Energiespeicherzustandsdaten festzulegen. Die Berücksichtigung des Zustands der Energiespeichereinheit bei der Festlegung der Ladeobergrenze und/oder des Alarmsignalschwellwerts und/oder der Ladespannung und/oder des Ladestroms kann vorteilhaft sein, um Einschränkungen bei der Benutzung der Steuereinrichtung möglichst gering zu halten.

In einer Variante dieser Ausführungsform können die Energiespeicherzustandsdaten eine aktuelle Maximalkapazität der Energiespeichereinheit umfassen. Ferner kann die Lademanagementeinheit ausgebildet sein, die Ladeobergrenze und/oder den Alarmsignalschwellwert relativ zur aktuellen Maximalkapazität festzulegen. Zusätzlich oder alternativ hierzu können die Energiespeicherzustandsdaten einen Speichergesundheitszustand umfassen und die Lademanagementeinheit ausgebildet sein, den Wert der Ladeleistung in Abhängigkeit von dem Speichergesundheitszustand festzulegen.

In einer anderen Ausführungsform der Steuereinrichtung kann zusätzlich oder alternativ zu den Merkmalen der zuvor beschriebenen, optionalen Ausführungsformen die mindestens eine Betriebsparametererfassungseinheit eine Eingabeschnittstelle umfassen, die ausgebildet ist, den Betriebsparameter anhand einer Benutzereingabe zu ermitteln. Zusätzlich kann in dieser Ausführungsform die Lademanagementeinheit ausgebildet sein, die Ladeobergrenze und/oder den Alarmsignalschwellwert und/oder die Ladespannung und/oder den Ladestrom in Abhängigkeit von der Benutzereingabe festzulegen. Die Möglichkeit die Ladeobergrenze und/oder den Alarmsignalschwellwert und/oder die Ladespannung und/oder den Ladestrom durch einen Benutzer wählen zu lassen, kann es ermöglichen, die Steuereinrichtung mehr an die Bedürfnisse des Benutzers anpassbar zu gestalten.

Bei einen weiteren Ausführungsform kann zusätzlich oder alternativ zu den Merkmalen der zuvor beschriebenen Ausführungsformen die Lademanagementeinheit ausgebildet sein, die elektrische Ladeleistung an der Energiespeichereinheit nur bereitzustellen, solange der Füllstand unterhalb der Ladeobergrenze liegt, und/oder die Lademanagementeinheit ausgebildet sein, das Alarmsignal bei einem Absinken des Füllstandes unterhalb des Alarmsignalschwellwertes bereitzustellen.

In einer anderen Ausführungsform kann zusätzlich oder alternativ die Lademanagementeinheit ausgebildet sein, den Wert der Ladespannung und/oder des Ladestroms durch Auswahl aus einer Vielzahl von vordefinierten Werteniveaus in Abhängigkeit des mindestens einen Betriebsparameters festzulegen.

In einer anderen Ausführungsform kann zusätzlich oder alternativ die Lademanagementeinheit ausgebildet sein, den Wert der Ladespannung und/oder des Ladestroms so festzulegen, dass dieser in Abhängigkeit des mindestens einen Betriebsparameters zeitabhängig zwischen zwei Extremwerten variiert. Dieser Ansatz entspricht einer Pulsweitenmodulation und ist eine Möglichkeit, eine mittlere Ladespannung und/oder einen Ladestrom in Abhängigkeit des mindestens einen Betriebsparameters anzupassen.

In Varianten dieser Ausführungsform kann die Lademanagementeinheit ausgebildet sein, den Wert der Ladeleistung periodisch, vorzugsweise in Form einer Rechteckfunktion, zu variieren und eine Verweildauer des Wertes der Ladeleistung bei dem jeweiligen Ladeleistungsextremum in Abhängigkeit von dem mindestens einen Betriebsparameter zu bestimmen.

Bei einer Ausführungsform kann es sich bei der Ladeschnittstelle um einen Ladeanschluss handeln. Der Ladeanschluss kann dabei einen Steckverbinder umfassen, der einen Anschluss der externen Energiequelle über ein Ladekabel ermöglicht. Dies kann beispielsweise vorgesehen sein für die Verwendung der Steuereinrichtung als extrakorporale Steuereinrichtung, d.h. eine Steuereinrichtung die sich während des Gebrauchs außerhalb des Körpers des Patienten befindet. Hierbei wird die Steuereinrichtung mittels eines Verbindungskabels (engl. Driveline), das durch eine Durchtrittsstelle in der Haut in den Körper des Patienten geführt wird, mit einer implantierbaren Blutpumpe elektrisch verbunden.

In einer hierzu alternativen Ausführungsform kann die Ladeschnittstelle ausgebildet sein, elektrische Energie kabellos zu empfangen. In diesem Fall kann die Steuereinrichtung beispielsweise eine implantierbare Steuereinrichtung sein. Durch die Ladeschnittstelle kann die implantierte Steuereinrichtung so von einer Transkutanen-Energie-Transfer (TET) Vorrichtung elektrische Energie durch Gewebe des Patienten hindurch empfangen. Die Ladeschnittstelle kann hierzu beispielsweise ausgebildet sein, elektrische Energie mittels Induktion zu empfangen.

In einer anderen Ausführungsform kann die Steuereinrichtung zusätzlich oder alternativ zu den zuvor im Zusammenhang mit den anderen Ausführungsformen beschriebenen Merkmalen ein Bestandteil einer Blutpumpeneinrichtung sein, die neben der Steuereinrichtung auch eine Blutpumpe und vorzugsweise ein Verbindungskabel (Driveline) zum Herstellen einer elektrischen Verbindung zwischen Blutpumpe und Steuereinrichtung umfasst.

In einer weiteren Ausführungsform kann es sich bei der Energiespeichereinheit um einen Akkumulator handeln. In Varianten dieser Ausführungsform kann es sich bei dem Akkumulator um einen Lithium-Ionen-Akku handeln.

Abschließend wird im Folgenden nun noch das Verfahren zum Betreiben einer Steuereinrichtung für eine Blutpumpe beschrieben. Dieses Verfahren umfasst die folgenden Schritte:
- Bereitstellen von elektrischer Energie mittels einer Energiespeichereinheit der Steuereinrichtung an einem Blutpumpenanschluss der Steuereinrichtung für einen Betrieb der Blutpumpe,
- Empfangen von elektrischer Energie an einer Ladeschnittstelle der Steuereinrichtung und Bereitstellen der elektrischen Energie in Form einer Ladeleistung an der Energiespeichereinheit zum Aufladen der Energiespeichereinheit, und
- Ermitteln mindestens eines Betriebsparameters der Steuer-einrichtung.
Zudem umfasst das Verfahren mindestens einen der folgenden Verfahrensschritte:
- Festlegen eines Wertes einer Ladespannung und/oder eines Wertes eines Ladestroms für die an der Energiespeichereinheit bereitgestellte Ladeleistung unter Benutzung eines Füllstandes der Energiespeichereinheit und mindestens einem der Betriebsparameter, und/oder
- Festlegen einer Ladeobergrenze, bis zu der die elektrische Energiespeichereinheit aufgeladen wird, unter Benutzung von mindestens einem der Betriebsparameter, und/oder
- Festlegen eines Alarmsignalschwellwertes, in Abhängigkeit dessen ein Alarmsignal bereitgestellt wird, unter Benutzung von mindestens einem der Betriebsparameter.

Weitere Ausführungsbeispiele der Steuereinrichtung und des Verfahrens zum Betreiben der Steuereinrichtung werden im Folgenden anhand der Figuren erklärt. Hierzu wird zunächst eine Übersicht über die Figuren gegeben.
Fig. 1 zeigt eine Steuereinrichtung für eine Blutpumpe;
Fig. 2 zeigt ein Diagramm über einen zeitlichen Verlauf eines Wertes einer Ladeleistung, mittels der eine Energiespeichereinheit der Steuereinrichtung aus Fig. 1 aufgeladen wird;
Fig. 3 zeigt eine alternative Ausführungsform einer Steuereinrichtung für eine Blutpumpe;
Fig. 4A zeigt eine Ladeobergrenze und einen Alarmsignalschwellwert im Fall einer am Blutpumpenanschluss der in Fig. 3 gezeigten Steuereinrichtung angeschlossenen Blutpumpe;
Fig. 4B zeigt die Ladeobergrenze und den Alarmsignalschwellwert in dem Fall, dass am Blutpumpenanschluss der in Fig. 3 gezeigten Steuereinrichtung keine Blutpumpe angeschlossen ist;
Fig. 5 zeigt eine Blutpumpeneinrichtung, die eine Blutpumpe und die Steuereinrichtung aus Fig. 1 umfasst; und
Fig. 6 zeigt ein Ausführungsbeispiel eines Verfahrens zum Betreiben einer Steuereinrichtung für eine Blutpumpe.

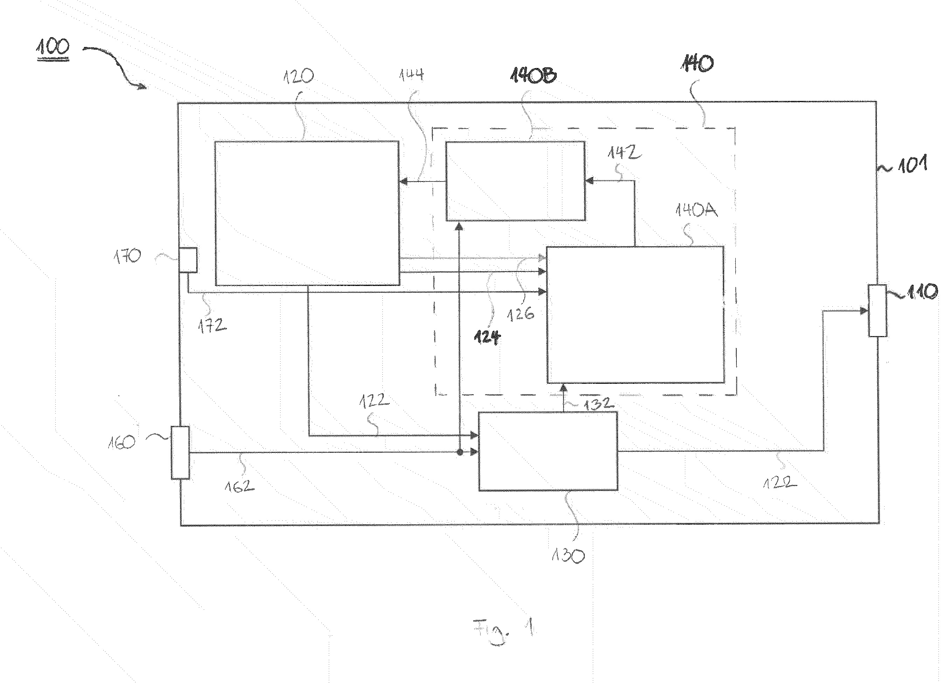

Im Folgenden werden die in den Figuren gezeigten Ausführungsbeispiele im Detail beschrieben. Ein erstes Ausführungsbeispiel wird zunächst anhand der Fign. 1, 2 und 5 beschrieben.

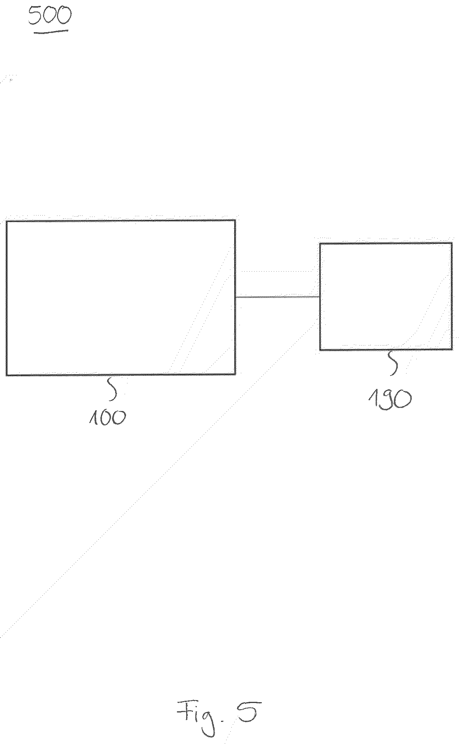

Fig. 1 zeigt eine Steuereinrichtung 100 für eine Blutpumpe 190. Fig. 5 zeigt eine Blutpumpeneinrichtung 500, die die Steuereinrichtung 100 zusammen mit der Blutpumpe 190 umfasst.

Die Steuereinrichtung 100 ist ausgebildet, elektrische Energie zum Betrieb der Blutpumpe 190 bereitzustellen. Hierzu umfasst die Steuereinrichtung 100 einen Blutpumpenanschluss 110 zur Herstellung einer elektrischen Verbindung zwischen der Steuereinrichtung 100 und der Blutpumpe 190, eine Energiespeichereinheit 120 zum Bereitstellen elektrischer Energie und eine Energiebereitstellungseinheit 130. Die Energiebereitstellungseinheit 130 ist ausgebildet, von der Energiespeichereinheit 120 für einen Betrieb der Blutpumpe 190 bereitgestellte elektrische Energie 122 von der Energiespeichereinheit 120 zu empfangen und diese am Blutpumpenanschluss 110 zur Verfügung zu stellen. Bei dem in Fig. 1 gezeigten Beispiel der Steuereinrichtung handelt es sich bei der Energiespeichereinheit 120 um einen Lithium-lonen-Akku. Prinzipiell kann aber auch ein anderer Akku mit der Steuereinrichtung verwendet werden.

Ferner umfasst die Steuereinrichtung 100 als Ladeschnittstelle einen Ladeanschluss 160 mittels dessen die Steuereinrichtung 100 an eine externe elektrische Energiequelle über ein Ladekabel anschließbar ist und eine Lademanagementeinheit 140. Die Lademanagementeinheit 140 ist ausgebildet, einen Füllstand 124 der Energiespeichereinheit 120 zu empfangen und zum Aufladen der Energiespeichereinheit 120 über den Ladeanschluss 160 empfangene elektrische Energie 162 an der Energiespeichereinheit 120 bereitzustellen. Die Lademanagementeinheit 140 kann unterschiedlich implementiert werden. In dem in Fig. 1 gezeigten Ausführungsbeispiel umfasst die Lademanagementeinheit 140 einen Mikrocontroller 140A und eine Ladeschaltung 140B. Der Mikrokontroller 140A ist dabei ausgebildet, den Füllstand 124 der Energiespeichereinheit 120 zu empfangen und einen Wert 142 einer an der Energiespeichereinheit 120 bereitgestellten Ladeleistung 144 festzulegen und an die Ladeschaltung 140B zu übermitteln. Die Ladeschaltung 140B ist wiederrum ausgebildet, den Wert der Ladeleistung 142 und eine am Ladeanschluss 160 bereitgestellte Energie zu empfangen und entsprechend eine Ladeleistung 144 an der Energiespeichereinheit 120 bereitzustellen.

Die in Fig. 1 gezeigte Steuereinrichtung 100 ist auch in der Lage, Steuersignale an die Blutpumpe 190 zu senden. In einigen Ausführungsbeispielen kann der Mikrokontroller 140A dabei sowohl alle für die Bereitstellung der Ladeleistung nötigen Funktionseinheiten als auch für die Bereitstellung der Steuersignale benötigten Funktionseinheiten umfassen. In anderen Ausführungsbeispielen können die Funktionsgruppen jedoch auch in unterschiedlichen Mikrokontrollern untergebracht sein. Dies soll an dieser Stelle aber nicht weiter thematisiert werden.

Ferner umfasst die Steuereinrichtung 100 mehrere Betriebsparametererfassungseinheiten, die ausgebildet sind, einen Betriebsparameter der Steuereinrichtung 100 zu ermitteln. Zudem ist die Lademanagementeinheit 140 ausgebildet, einen Wert 142 der Ladeleistung für die an der Energiespeichereinheit 120 bereitgestellte Ladeleistung 142 unter Benutzung des Füllstands 124 und mindestens einem der Betriebsparameter festzulegen.

Bei dem in Fig. 1 gezeigten Ausführungsbeispiel handelt es sich bei der Betriebsparametererfassungseinheit um eine Temperatursensoreinheit 170, die ausgebildet ist, als Betriebsparameter eine Umgebungstemperatur 172 am Ort der Temperatursensoreinheit 170 zu ermitteln.

In dem gezeigten Ausführungsbeispiel ist die Temperatursensoreinheit 170 zwischen Energiespeichereinheit 120 und einer zur Energiespeichereinheit 120 nächsten Gehäuseoberfläche eines Gehäuses 101 der Steuereinrichtung 100 angeordnet. Ferner ist die Abhängigkeit zwischen dem Wert 142 der Ladeleistung und der Umgebungstemperatur 172 so gewählt sein, dass der Wert 142 der Ladeleistung umso geringer ist, je größer die Umgebungstemperatur 172 ist, um ein Überschreiten einer kritischen Temperatur an der Oberfläche der Steuereinrichtung zu verhindern. Die gezeigte Anordnung der Temperatursensoreinheit 170 ist jedoch nur ein Beispiel. In anderen Ausführungsbeispielen kann die Temperatursensoreinheit auch an einem anderen Ort innerhalb oder an der Steuereinrichtung angeordnet sein.

Zusätzlich zu der Temperatursensoreinheit 170 umfasst die Energiebereitstellungseinheit 130 als weitere Betriebsparametererfassungseinheit eine Ladequellenerkennungseinheit, die ausgebildet ist, als einen weiteren Betriebsparameter einen Ladequellentyp 132 einer an den Ladeanschluss 160 angeschlossenen elektrischen Energiequelle zu ermitteln. Ferner ist die Lademanagementeinheit 140 ausgebildet, den Wert 142 der Ladeleistung in Abhängigkeit von dem Ladequellentyp 132 festzulegen. Das Ermitteln des Ladequellentyps kann auf verschiedene Weise erfolgen. Im gezeigten Ausführungsbeispiel ist die Energiebereitstellungseinheit 130 ausgebildet, den Ladequellentype 132 anhand einer am Ladeanschluss zur Verfügung gestellten Ladespannung zu ermitteln.

In dem Ausführungsbeispiel kann die Ladequellenerkennungseinheit beispielsweise unterscheiden, ob die Steuereinrichtung 100 über den Ladeanschluss 170 an eine Netzversorgung oder an eine externe Energiespeichereinheit angeschlossen ist. Ferner ist der Mikrokontroller 140A ausgebildet, den Wert der Ladeleistung in Abhängigkeit vom empfangenen Ladequellentyp 132 festzulegen. Hierdurch können der Ladequelle entsprechende Ladeleistungen 144 für das Aufladen der Energiespeichereinheit bereitgestellt werden, beispielsweise eine höhere Ladeleistung beim Anschluss an eine Netzversorgung als beim Anschluss an eine externe Energiespeichereinheit. Beim Anschluss einer externen Energiespeichereinheit ist es auch oft vorteilhaft, zunächst die durch die externe Energiespeichereinheit bereitgestellte Leistung mehrheitlich zum Betrieb der Blutpumpe zu verwenden. Hierdurch kann verhindert werden, dass die externe Energiespeichereinheit schnell geleert wird und der Nutzer bereits nach kurzer Zeit zum Austausch des externen Akkus aufgefordert wird. Auch diese Überlegungen können in den Mikrokontroller 140A umgesetzte Abhängigkeit zwischen Ladequellentyp 132 und Wert 142 der Ladeleistung einfließen.

Zudem umfasst in dem in Fig. 1 gezeigten Ausführungsbeispiel die Energiespeichereinheit 120 selbst als weitere Betriebsparametererfassungseinheit eine Energiespeicherzustandsermittlungseinheit, die ausgebildet ist, als einen Betriebsparameter Energiespeicherzustandsdaten 126 in Abhängigkeit eines Zustands der Energiespeichereinheit 120 bereitzustellen. Zudem ist der Mikrokontroller 140A der Lademanagementeinheit 140 ausgebildet, den Wert 142 der Ladeleistung in Abhängigkeit von den Energiespeicherzustandsdaten 126 festzulegen. Dabei umfassen die Energiespeicherzustandsdaten 126 einen Speichergesundheitszustand, der ein Indikator für einen Verschleiß der Energiespeichereinheit 126 ist. Außerdem ist der Mikrokontroller 140A der Lademanagementeinheit 140 ausgebildet, den Wert 142 der Ladeleistung umso geringer zu wählen, je schlechter der Speichergesundheitszustand ist.

Bei der in Fig. 1 gezeigten Steuereinrichtung 100 wird der Wert der Ladeleistung in Abhängigkeit des Füllstands 124 und mindestens einem der Betriebsparameter 126, 132, 172 festgelegt. Der Mikrokontroller 140A kann dabei so ausgebildet sein, dass der Wert der Ladeleistung entsprechend den Betriebsparametern erhöht oder abgesenkt wird. Dies kann beispielsweise durch Auswahl aus einer Vielzahl von vordefinierten Werteniveaus in Abhängigkeit eines oder mehrerer Betriebsparameters erfolgen.

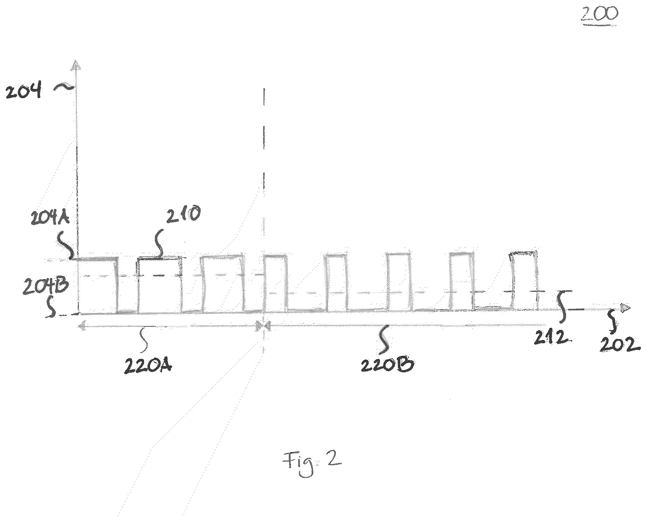

Alternativ hierzu ist es jedoch auch möglich, die Ladeleistung mittels einer Pulsweitenmodulation vorzugeben. Dies wird im Folgenden mit Bezugnahme auf Fig. 2 vertieft.

Fig. 2 zeigt ein Diagramm 200 über einen zeitlichen Verlauf 210 des Wertes einer Ladeleistung, mittels der die Energiespeichereinheit 120 der Steuereinrichtung aus Fig. 1 aufgeladen wird.

Im Diagramm 200 ist der Wert der Ladeleistung auf einer Amplitudenachse 204 als Verlauf über einer Zeitachse 202 dargestellt. Der Wert der Ladeleistung variiert dabei über der Zeit in Form einer Rechteckfunktion zwischen einem Maximalwert 204A und einem Minimalwert 204B, in diesem Fall Null. Im Diagramm ist dabei zu erkennen, dass in einem ersten Zeitabschnitt 220A der Wert der Ladeleistung im Schnitt für 2/3 der Zeit einer Periode der Rechteckfunktion bei dem Maximalwert 204A liegt und nur in einem 1/3 der Zeit beim Minimalwert 204B. Ein Mittelwert 212 der Amplitude liegt dadurch bei 2/3 des Maximalwertes 204A. In einem zweiten Zeitabschnitt 220B liegt der Wert der Ladeleistung im Mittel nur bei 1/3 des Maximalwertes. Entsprechend der Wahl der Zeitdauern, bei denen die Ladeleistung bei dem Maximalwert bzw. Minimalwert liegt, lassen sich so beliebige, durchschnittliche Ladeleistungen zwischen dem Maximalwert und dem Minimalwert einstellen.

Bei dem hier beschrieben Ausführungsbeispiel wird der Wert 142 der Ladeleistung durch die Lademanagementeinheit 140 festgelegt. In anderen Ausführungsbeispielen kann aber auch ein Wert entweder einer Ladespannung oder eines Ladestroms vorgegeben sein und nur der entsprechende andere Wert variiert werden.

In dem gezeigten Beispiel der Steuereinrichtung 100 werden verschiedene Umgebungsparameter gemeinsam genutzt, um den Wert 142 der Ladeleistung festzulegen. Grundsätzlich ist es aber auch möglich eine beliebige Auswahl dieser oder auch anderer Umgebungsparameter zu verwenden.

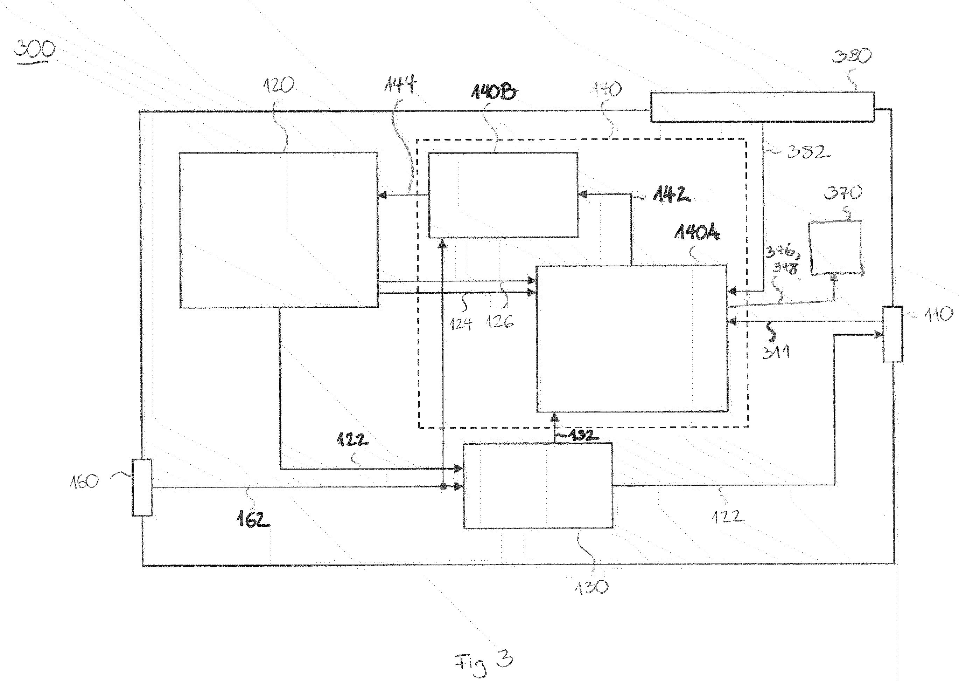

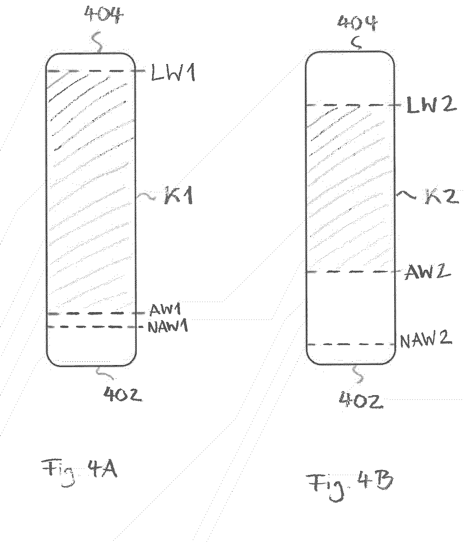

Alternativ oder zusätzlich zum Festlegen der Ladeleistung können Umgebungsparameter auch zum Festlegen anderer, für das Laden der Energiespeichereinheit relevante Parameter verwendet werden. Ein entsprechendes Ausführungsbeispiels wird im Folgenden unter Bezugnahme zu Fign. 3 und 4 beschrieben.

Fig. 3 zeigt ein alternatives Ausführungsbeispiel einer Steuereinrichtung 300 für Blutpumpe.

Das in Fig. 3 gezeigte Ausführungsbeispiel ist weitestgehend identisch zu dem in Fig. 1 gezeigten Ausführungsbeispiel. Alle bereits in Fig. 1 gezeigten Komponenten sind in Fig. 3 mit gleichem Bezugszeichen versehen und werden im Folgenden nur beschrieben, wenn sich deren Funktionsweise zu dem in Fig. 1 gezeigten Ausführungsbeispiel unterscheidet.

Ein Unterschied zum Ausbildungsbeispiel der Fig. 1 ist das die Lademanagementeinheit 140 der Steuereinrichtung 300 ausgebildet ist, eine Ladeobergrenze, bis zu der die Energiespeichereinheit 120 geladen wird, unter Benutzung von Betriebsparametern festzulegen, sowie einen Alarmsignalschwellwert, in Abhängigkeit dessen ein Alarmsignal 346 bereitgestellt wird, unter Benutzung von mindestens einem der Betriebsparameter festzulegen. Die Lademanagementeinheit 140 ist ferner ausgebildet, die elektrische Ladeleistung an der Energiespeichereinheit 120 nur bereitzustellen, solange der Füllstand der Energiespeichereinheit 120 unterhalb der Ladeobergrenze liegt. Weiterhin ist die Lademanagementeinheit 140 ausgebildet, das Alarmsignal 346 bereitzustellen, wenn der Füllstand der Energiespeichereinheit 120 den Alarmsignalschwellwert unterschreitet. Bei dem konkreten Ausführungsbeispiel der Steuereinrichtung 300 ist der Mikrokontroller 140A ausgebildet, den Füllstand 124 der Energiespeichereinheit 120 in regelmäßigen Abständen mit der Ladeobergrenze und dem Alarmsignalschwellwert abzugleichen. Überschreitet der Füllstand 124 die Ladeobergrenze, ist der Mikrokontroller 140A ausgebildet, die Ladeleistung 142 auf null zu reduzieren, so dass die Ladeschaltung 140B die Energiespeichereinheit 120 nicht weiter lädt. Sollte der Füllstand 124 den Alarmsignalschwellwert unterschreiten, ist der Mikrokontroller 140A ausgebildet, das Alarmsignal 346 bereitzustellen. Das Alarmsignal 346 wird dann an eine Alarmeinheit 370 weitergeleitet, die in Abhängigkeit von dem Alarmsignal 142 ein akustisches Warnsignal ausgibt. Die Ausgabe des akustischen Warnsignals ist hierbei jedoch nur als ein Beispiel zu verstehen. In anderen Ausführungsbeispielen kann das Warnsignal auch in anderer Form an einen Benutzer der Steuereinrichtung weitergegebene werden. Die Ladeobergrenze und der Alarmsignalschwellwert können dabei beispielsweise anhand einer Spannung, d.h. einer Ladeschlussspannung bzw. einer Alarmsignalschwellspannung der Energiespeichereinheit 120, realisiert werden.

Im Stand der Technik ist bereits bekannt, dass ein wiederholtes vollständiges Laden und ein vollständiges Entladen von Energiespeichereinheiten bestimmter Typen, wie beispielsweise Lithium-lonen-Akkus, zu einer Reduzierung einer Speicherkapazität der elektrischen Energiespeichereinheit 120 führen kann. Diesem soll durch das Festlegen der Ladeobergrenze und des Alarmsignalschwellwertes in Abhängigkeit von Umgebungsparametern entgegengewirkt werden.

Ein generelles Reduzieren der Ladeobergrenze der Energiespeichereinheit 120 sowie eine generelle Erhöhung des Alarmsignalschwellwertes für die Energiespeichereinheit 120 kann dazu genutzt werden, zu verhindern, dass die Energiespeichereinheit 120 immer vollständig geladen bzw. fast vollständig entladen wird. Dies kann einer Reduzierung der Lebensdauer der Energiespeichereinheit 120 entgegenwirken. Allerdings hat dies einen großen, negativen Einfluss auf die Benutzbarkeit der Steuereinrichtung 300 der Blutpumpe, weil die Steuereinrichtung 300 dann häufiger geladen werden muss. Um diesem negativen Einfluss entgegenzuwirken, umfasst die Steuereinrichtung 300 eine Mehrzahl von Betriebsparametererfassungseinheiten, um die Ladeobergrenze nur dann zu reduzieren bzw. den Alarmsignalschwellwert nur dann zu erhöhen, wenn dies das Benutzen der Steuereinrichtung nur wenig beeinflusst.

Eine Situation, bei der eine Einschränkung des Ladens bzw. des Entladens der Energiespeichereinheit 120 nur wenig Einfluss auf den Benutzer hat, ist, wenn entweder die Blutpumpe gar nicht am Blutpumpenanschluss 110 angeschlossen ist oder die benötigte elektrische Leistung der Blutpumpe nur gering ist. Dies kann beispielsweise der Fall sein, wenn die Steuereinrichtung als Ersatzgerät fungiert und erst eingesetzt wird, wenn eine aktuell verwendete Steuereinrichtung aufgrund beispielsweise eines Defekts ausgetauscht werden muss. Um die Ladeobergrenze und die Alarmsignalschwelle nur in Momenten anzupassen, wenn die Leistungsabgabe am Blutpumpenladeanschluss gering ist, umfasst die Steuereinrichtung 300 einen Blutpumpenanschluss 110, der eine Betriebsparametererfassungseinheit aufweist, die ausgebildet ist, festzustellen, ob eine Blutpumpe am Blutpumpenanschluss angeschlossen ist und in Abhängigkeit davon Leistungsabgabedaten 110 an der Lademanagementeinheit bereitzustellen. Die Lademanagementeinheit 140 ist weiterhin ausgebildet, sowohl die Ladeobergrenze als auch den Alarmsignalschwellwert in Abhängigkeit von den Leistungsabgabedaten festzulegen. Um festzustellen, ob eine Blutpumpe am Blutpumpenanschluss 110 angeschlossen ist, kann beispielsweise eine Widerstands- oder Spannungsmessung verwendet werden. Auch ist es möglich, einen separaten Pin zu Detektion der Blutpumpe zu verwenden.

In anderen Ausführungsbeispielen kann alternativ oder zusätzlich hierzu die Energiebereitstellungseinheit 130, eine Betriebsparametererfassungseinrichtung umfassen, die ausgebildet ist, eine an der Blutpumpenanschluss 110 abgeführte elektrische Leistung zu bestimmen und in Abhängigkeit davon die Leistungsabgabedaten an der Lademanagementeinheit 140 bereitzustellen.

Auch kann es sinnvoll sein, beim Laden der Energiespeichereinheit 120 zu berücksichtigen, welche Art von externer Energiequelle verwendet wird, um die Energiespeichereinheit 120 zu laden. Wird beispielsweise an den Ladeanschluss 160 ebenfalls eine Energiespeichereinheit angeschlossen, deren Lebensdauer durch ein vollständiges Entladen negativ beeinflusst wird, kann es sinnvoll sein, auch beim Festlegen der Ladeobergrenze den Ladequellentyp zu berücksichtigen. Wie bereits bei der Steuereinrichtung 100 beschrieben, umfasst die die Energiebereitstellungseinheit 130 der Steuereinrichtung 300 deshalb als eine weitere Betriebsparametererfassungseinheit eine Ladequellenerkennungseinheit, die ausgebildet ist, als einen weiteren Betriebsparameter einen Ladequellentyp 132 einer an den Ladeanschluss 160 angeschlossenen elektrischen Energiequelle zu ermitteln. Ferner ist die Lademanagementeinheit 140 ausgebildet, den Wert 142 der Ladeobergrenze in Abhängigkeit des Ladequellentyps 132 festzulegen. Die Abhängigkeit zwischen Ladequellentyp und Ladeobergrenzen kann dabei verschieden ausgestaltet sein. In dem hier gezeigten Ausführungsbeispiel wird die Ladeobergrenze niedriger gewählt, wenn es sich bei der externen Energiequelle um eine externe Energiespeichereinheit handelt. In anderen Ausführungsformen kann zusätzlich hierzu auch noch eine Speicherkapazität der externen Energiequelle berücksichtigt werden bzw. zwischen verschiedenen Arten von externen Energiespeichereinheiten unterschieden werden.

Gerade wenn durch das Setzen einer reduzierten Ladeobergrenze und/oder eines erhöhten Alarmsignalschwellwertes die nutzbare Kapazität der Energiespeichereinheit 120 effektiv reduzierten wird, sollte es auch eine Möglichkeit geben, dass der Benutzer Einfluss auf diese Einstellungen hat. Dies ist beispielsweise dann sinnvoll, wenn der Benutzer bereits weiß, dass ein längerer Ausflug geplant ist und ein Aufladen der Energiespeichereinheit 120 für eine bestimmte Zeit nicht möglich ist. Für dieses Fall umfasst die Steuereinrichtung 300 zusätzlich über eine Eingabeschnittstelle 380 als zusätzliche Betriebsparametererfassungseinheit, die ausgebildet ist, einen Betriebsparameter 382 anhand einer Benutzereingabe zu ermitteln. Zudem ist die Lademanagementeinheit 140 ausgebildet ist, die Ladeobergrenze und den Alarmsignalschwellwert in Abhängigkeit von der Benutzereingabe festzulegen. Die Benutzereingabe kann dabei beispielsweise über die Auswahl eines Betriebsmodus erfolgen. Möglich wären beispielsweise ein Eco-Modus, der mit einem reduzierten Laden und Entladen der Energiespeichereinheit 120 verbunden ist, und ein Mobilitätsmodus, bei dem die volle Speicherkapazität der Energiespeichereinheit 120 genutzt wird.

Anhand der Fign. 4A und 4B soll im Folgenden beispielhaft beschrieben werden, wie die Anpassung der Ladeobergrenze und des Alarmsignalschwellwertes bei der Steuereinrichtung 300 erfolgt.

Fig. 4A zeigt die Ladeobergrenze und den Alarmsignalschwellwert im Fall einer am Blutpumpenanschluss 110 angeschlossenen Blutpumpe. Fig. 4B zeigt die Ladeobergrenze und den Alarmsignalschwellwert in dem Fall, dass am Blutpumpenanschluss 110 keine Blutpumpe angeschlossen ist. Fign. 4A und 4B stellen dabei eine Illustration eines Füllstandes der Energiespeichereinheit 120 da, bei der eine untere Grenze 402 eine vollkommene Entladung der Energiespeichereinheit 120 repräsentiert und eine obere Grenze 404 eine vollkommen geladene Energiespeichereinheit 120. Die Ladeobergrenze und der Alarmsignalschwellwert können zwischen diesen beiden Grenzen variiert werden.

Wie bereits oben beschrieben, ist der Blutpumpenanschluss 110 ausgebildet, Leistungsabgabedaten 311 in Abhängigkeit davon bereitzustellen, ob eine Blutpumpe am Blutpumpenanschluss 110 angeschlossen ist. In dem Fall, dass eine Blutpumpe angeschlossen ist, muss davon ausgegangen werden, dass die Leistungsabgabe an die Blutpumpe zu einer schnellen Reduktion des Füllstandes des Energiespeichers führen wird. Damit die Steuereinrichtung 300 über eine längere Zeit genutzt werden kann, ist die Lademanagementeinheit 140 ausgebildet, in diesem Fall die Ladeobergrenze auf einen Wert LW1 zu setzen und den Alarmsignalschwellwert auf einen Wert AW1 zu setzen, die beide sehr nahe an den jeweiligen Grenzen 402 und 404 der Energiespeichereinheit 120 liegen. Bei einem Laden der Energiespeichereinheit 120 bis zur Ladeobergrenze LW1 und einem Entladen bis zum Alarmsignalschwellwert AW1 steht eine große verfügbare Speicherkapazität K1 zur Verfügung, die durch den straffierten Bereich illustriert ist. Weil die Ladeobergrenze LW1 aber sehr nahe an der oberen Grenze 404 liegt und der Alarmsignalschwellwert AW1 an der unteren Grenze 402 liegt, kann ein wiederholtes Laden bzw. ein Entladen der Energiespeichereinheit 120 bis zu diesen Grenzen jedoch die Gesamtspeicherkapazität der Energiespeichereinheit 120 über die Zeit reduzieren.

Aus diesem Grund ist die Lademanagementeinheit 140 ausgebildet, die Ladeobergrenze auf einen Wert LW2 herabzusenken und den Alarmsignalschwellwert auf einen Wert AW2 heraufzusetzen, wenn keine Blutpumpe am Blutpumpenanschluss 110 angeschlossen ist. Dieser Fall ist in Fig. 4B illustriert. Dies führt zwar zu einer verringerten nutzbaren Kapazität K2. Da die Leistungsabgabe der Energiespeichereinheit 120 jedoch auch reduziert ist, kann die Steuereinrichtung 300 vergleichbar lange wie im in Fig. 4A illustrierten Fall betrieben werden oder vielleicht sogar länger. Gleichzeitig wird die Energiespeichereinheit geschont, so dass die Speicherkapazität der Energiespeichereinheit 120 auch bei wiederholtem Laden und Entladen sich weniger stark reduziert.

Die Lademanagementeinheit 140 ist dabei ausgebildet, die Ladeobergrenze und den Alarmsignalschwellwert in Abhängigkeit einer Maximalkapazität der Energiespeichereinheit 120, einer zu erwartenden Leistungsaufnahme der Steuereinrichtung 300, die anhand der Leistungsabgabedaten 311 ermittelbar sind, und einer Mindestbetriebsdauer festzulegen. In einigen Ausbildungsformen der Steuereinrichtung ist die Lademanagementeinheit 140 zusätzlich ausgebildet, bei der Festlegung der Ladeobergrenze und des Alarmsignalschwellwertes einen Zustand der Energiespeichereinheit 120 zu berücksichtigen. So kann beispielsweise beim Festlegen der Ladeobergrenze und des Alarmsignalschwellwertes eine aktuelle Maximalkapazität der Energiespeichereinheit berücksichtigt werden, so dass auch bei durch Verschleiß herabgesetzter Maximalkapazität eine nutzbare Kapazität entsprechend der am Blutpumpenanschluss 110 zur Verfügung zu stellenden Leistung bereitgestellt werden kann.

Zusätzlichen zu dem bereits beschriebenen Alarmsignalschwellwert können einige Ausführungsbeispiele auch ausgebildet sein, einen Notalarmsignalschwellwert festzulegen. Für den Benutzer der Steuereinrichtung kann es hilfreich sein, ein weiteres akustisches Warnsignal zu erhalten, wenn bei einer zu erwartenden Leistungsaufnahme eine vordefinierte Mindestrestlaufzeit der Steuereinrichtung unterschritten wird.

Die Lademanagementeinheit 140 der Steuereinrichtung 300 ist daher ausgebildet, neben der Ladeobergrenze und dem Alarmsignalschwellwert auch einen Wert des Notalarmsignalschwellwerts festzulegen. Dieser Wert wird so festgelegt, dass das Notalarmsignal ausgelöst wird, wenn bei einer zu erwartenden Leistungsaufnahme der Steuereinrichtung 300, die anhand der Leistungsabgabedaten 311 ermittelbar ist, eine vordefinierten Mindestrestlaufzeit der Steuereinrichtung 300 unterschritten wird. Ferner ist die Lademanagementeinheit 140 ausgebildet, bei einem Absinken des Füllstands der Energiespeichereinheit 120 unterhalb des Notalarmsignalschwellwerts ein Notalarmsignal 348 an die Alarmeinheit 370 bereitzustellen. Die Alarmeinheit 370 ist wiederum ausgebildet, in Abhängigkeit des Notalarmsignals 348 ein zweites akustisches Warnsignal auszugeben.

In dem Beispiel der Fig. 4A ist die Leistungsabgabe aufgrund der am Blutpumpenanschluss angeschlossenen Blutpumpe hoch. In dem Beispiel der Fig. 4B ist die Leistungsabgabe niedrig, da keine Blutpumpe an der Steuereinrichtung angeschlossen ist. In beiden Fällen ist die Mindestrestlaufzeit der Steuereinrichtung identisch. Durch die unterschiedliche Leistungsabgabe liegt der von der Lademanagementschaltung 140 festgelegte Notalarmsignalschwellwert NAW1 in Fig. 4A jedoch höher als der festgelegte Notalarmsignalschwellwert NAW2 in Fig. 4B.

Bei den Ausführungsbeispielen der Fig. 1 wird der Wert der Ladeleistung unter Verwendung mindestens eines Betriebsparameters festgelegt. Bei dem Ausführungsbeispiel der Fig. 3 wird die Ladeobergrenze und der Alarmsignalschwellwert unter Verwendung mindestens eines Betriebsparameters festgelegt. Dies ist jedoch nur beispielhaft zu verstehen. In anderen Ausführungsformen kann durch die Steuereinrichtung eine beliebige Auswahl dieser drei Grö-ßen festgelegt werden, d.h. nur eine der vier Größen, mehrere der vier Grö-ßen oder alle vier Größen.

Ferner wird in Fig. 1 und 3 eine Steuereinrichtung gezeigt, die außerhalb des Körpers eines Patienten getragen und über den Ladeanschluss kabelgebunden mit einer externen Energiequelle verbindbar ist. Genauso ist es jedoch auch möglich, dass die Steuereinrichtung anstatt eines Ladeanschlusses mit Steckverbinder auch eine Ladeschnittstelle aufweist, die ausgebildet ist, elektrische Energie kabellos über eine induktive Energieübertragung zu empfangen. Eine solche Steuereinrichtung kann zusätzlich implantierbar ausgebildet sein, so dass diese sich zusammen mit der Blutpumpe im Körper des Patienten implantiert werden kann und mittels eines transkutanen Energietransfers (TET) elektrische Energie von einer Ladevorrichtung außerhalb des Körpers empfängt. Der Ladequellentyp kann in dieser Ausführungsform bspw. dadurch ermittelt werden, dass die energiebereitstellende Vorrichtung entsprechende Ladequellentypdaten ebenfalls über die Ladeschnittstelle bereitstellt. Eine Umgebungsparametererfassungseinheit der Steuereinrichtung kann in diesem Fall ausgebildet sein, unter Verwendung der Ladequellentypdaten einen Ladequellentyp der externen Energiequelle zu ermitteln.

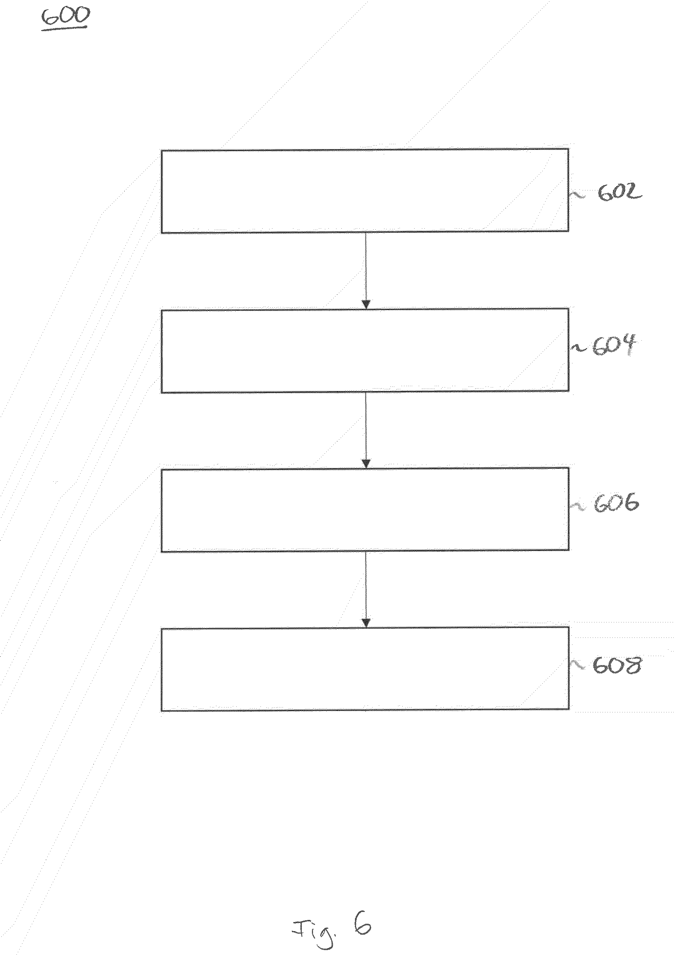

Abschließend soll anhand der Fig. 6 noch ein Verfahren zum Betreiben einer Steuereinrichtung für eine Blutpumpe beschrieben werden.

Fig. 6 zeigt einzelne Verfahrensschritten 602-608 eines Verfahrens 600 zum Betreiben der Steuereinrichtung.

Das Verfahren 600 beginnt mit einem Verfahrensschritt 602, der ein Bereitstellen von elektrischer Energie mittels einer Energiespeichereinheit der Steuereinrichtung an einem Blutpumpenanschluss der Steuereinrichtung für einen Betrieb der Blutpumpe umfasst.

Ein zweiter Verfahrensschritt 604 beinhaltet ein Empfangen von elektrischer Energie an einer Ladeschnittstelle der Steuereinrichtung und Bereitstellen der elektrischen Energie in Form einer Ladeleistung an der Energiespeichereinheit zum Aufladen der Energiespeichereinheit.

Ein weiterer Verfahrensschritt 606 umfasst ein Ermitteln mindestens eines Betriebsparameters der Steuereinrichtung.

In einem Verfahrensschritt 608 wird wahlweise:
- ein Wert einer Ladespannung und/oder ein Wert eines Ladestroms für die an der Energiespeichereinheit bereitgestellte Ladeleistung unter Benutzung eines Füllstandes der Energiespeichereinheit und mindestens einem der Betriebsparameter festgelegt,
- eine Ladeobergrenze, bis zu der die elektrische Energiespeichereinheit aufgeladen wird, unter Benutzung von mindestens einem der Betriebsparameter festgelegt, und/oder
- ein Alarmsignalschwellwert, in Abhängigkeit dessen ein Alarmsignal bereitgestellt wird, unter Benutzung von mindestens einem der Betriebsparameter festgelegt.

Wie bereits im Zusammenhang mit den Ausführungsbeispielen der Steuereinrichtung 100 und 300 erläutert, kann dabei in Verfahrensschritt 608 nur eine der Größen Ladespannung, Ladestroms, Ladeobergrenze oder Alarmsignalschwellwert festgelegt werden, mehrere dieser Größen oder auch alle diese Größen festgelegt werden.

Zusammenfassend betrifft diese Offenbarung eine Steuereinrichtung (100) für eine Blutpumpe (190), die einen Blutpumpenanschluss (110) zur Herstellung einer elektrischen Verbindung zwischen der Steuereinrichtung (100) und der Blutpumpe (190) umfasst. Zudem umfasst die Steuereinrichtung (100) eine Energiespeichereinheit (120) zum Bereitstellen elektrischer Energie sowie eine Energiebereitstellungseinheit (130), die ausgebildet ist, für einen Betrieb der Blutpumpe (190) von der Energiespeichereinheit (120) bereitgestellte elektrische Energie am Blutpumpenanschluss (110) zur Verfügung zu stellen. Ferner umfasst die Steuereinrichtung (300) eine Ladeschnittstelle (160), die ausgebildet ist, elektrische Energie einer externen elektrischen Energiequelle zu empfangen, und eine Lademanagementeinheit (140), die ausgebildet ist, einen Füllstand (124) der Energiespeichereinheit (120) zu empfangen und zum Aufladen der Energiespeichereinheit (120) über die Ladeschnittstelle (160) empfangene elektrische Ladeleistung an der Energiespeichereinheit (120) bereitzustellen. Zudem weist die Steuereinrichtung (100) mindestens eine Betriebsparametererfassungseinheit (170) auf, die ausgebildet ist, einen Betriebsparameter (172) der Steuereinrichtung (100) zu ermitteln. Weiterhin ist die Lademanagementeinheit (140) ausgebildet ist, einen Wert (142) einer Ladespannung, einen Wert (142) eines Ladestrom, eine Ladeobergrenze (LW1, LW2) und/oder einen Alarmsignalschwellwert (AW1, AW2) unter Benutzung von mindestens einem der Betriebsparameter (172) festzulegen.

## Patentansprüche

1. Steuereinrichtung (100) für eine Blutpumpe (190), umfassend:
einen Blutpumpenanschluss (110) zur Herstellung einer elektrischen Verbindung zwischen der Steuereinrichtung (100) und der Blutpumpe (190),
eine Energiespeichereinheit (120) zum Bereitstellen elektrischer Energie,
eine Energiebereitstellungseinheit (130), die ausgebildet ist, für einen Betrieb der Blutpumpe (190) von der Energiespeichereinheit (120) bereitgestellte elektrische Energie am Blutpumpenanschluss (110) zur Verfügung zu stellen,
eine Ladeschnittstelle (160), die ausgebildet ist, elektrische Energie einer externen elektrischen Energiequelle zu empfangen, und
eine Lademanagementeinheit (140), die ausgebildet ist, einen Füllstand (124) der Energiespeichereinheit (120) zu empfangen und zum Aufladen der Energiespeichereinheit (120) über die Ladeschnittstelle (160) empfangene elektrische Ladeleistung an der Energiespeichereinheit (120) bereitzustellen; wobei
die Steuereinrichtung (100) mindestens eine Betriebsparametererfassungseinheit (170) aufweist, die ausgebildet ist, einen Betriebsparameter (172) der Steuereinrichtung (100) zu ermitteln, und
die Lademanagementeinheit (140) ausgebildet ist, einen Wert (142) einer Ladespannung und/oder einen Wert (142) eines Ladestrom für die an der Energiespeichereinheit (120) bereitgestellte Ladeleistung unter Benutzung des Füllstands (124) und mindestens einem der Betriebsparameter (172) festzulegen, und/oder
die Lademanagementeinheit (140) ausgebildet ist, eine Ladeobergrenze (LW1, LW2), bis zu der die Energiespeichereinheit (120) geladen wird, unter Benutzung von mindestens einem der Betriebsparameter (311) festzulegen, und/oder
die Lademanagementeinheit (140) ausgebildet ist, einen Alarmsignalschwellwert (AW1, AW2), in Abhängigkeit dessen ein Alarmsignal (346) bereitgestellt wird, unter Benutzung von mindestens einem der Betriebsparameter (311) festzulegen.

2. Steuereinrichtung (100) nach einem der vorhergehenden Ansprüche, wobei
die mindestens eine Betriebsparametererfassungseinheit eine Temperatursensoreinheit (172) umfasst, die ausgebildet ist, als Betriebsparameter eine Umgebungstemperatur (172) am Ort der Temperatursensoreinheit zu ermitteln, und
die Lademanagementeinheit (140) ausgebildet ist, den Wert (142) der Ladespannung und/oder den Wert (142) des Ladestroms in Abhängigkeit von der Umgebungstemperatur (172) festzulegen.

3. Steuereinrichtung (100) nach einem der vorhergehenden Ansprüche, wobei
die mindestens eine Betriebsparametererfassungseinheit eine Ladequellenerkennungseinheit (130) umfasst, die ausgebildet ist, als einen Betriebsparameter einen Ladequellentyp (132) einer über die Ladeschnittstelle (160) Energie bereitstellenden externen elektrischen Energiequelle zu ermitteln, und
die Lademanagementeinheit (140) ausgebildet ist, den Wert (142) der Ladespannung und/oder den Wert (142) des Ladestroms in Abhängigkeit von dem Ladequellentyp (132) festzulegen.

4. Steuereinrichtung (300) nach einem der vorhergehenden Ansprüche, wobei
die mindestens eine Betriebsparametererfassungseinheit ausgebildet ist, als einen Betriebsparameter Leistungsabgabedaten (311) in Abhängigkeit von einer über den Blutpumpenanschluss (110) abgegebenen Leistung bereitzustellen, und
die Lademanagementeinheit (140) ausgebildet ist, die Ladeobergrenze (LW1, LW2) und/oder den Alarmsignalschwellwert (AW1, AW2) in Abhängigkeit von den Leistungsabgabedaten (311) festzulegen.

5. Steuereinrichtung (300) nach Anspruch 4, wobei die die Leistungsabgabedaten (311) bereitstellende Betriebsparametererfassungseinheit ausgebildet ist, die Leistungsabgabedaten (311) durch Messung einer elektrischen Leistung zu ermitteln.

6. Steuereinrichtung (300) nach Anspruch 4 oder 5, wobei die die Leistungsabgabedaten (311) bereitstellende Betriebsparametererfassungseinheit ausgebildet ist, die Leistungsabgabedaten (311) in Abhängigkeit davon bereitzustellen, ob eine Blutpumpe (190) am Blutpumpenanschluss (110) angeschlossen ist.

7. Steuereinrichtung (300) nach einem der Ansprüche 4 bis 6, wobei die Lademanagementeinheit (140) ausgebildet ist, den Alarmsignalschwellwert (AW1, AW2) und/oder die Ladeobergrenze (LW1, LW2) in Abhängigkeit einer Maximalkapazität der Energiespeichereinheit (120) und der Leistungsabgabedaten (311) festzulegen.

8. Steuereinrichtung (300) nach Anspruch 7, wobei die Lademanagementeinheit (140) ausgebildet ist, den Alarmsignalschwellwert (AW1, AW2) unter Benutzung der Leistungsabgabedaten (311) und einer gegebenen Mindestrestbetriebsdauer der Steuereinrichtung (300) festzulegen.

9. Steuereinrichtung (100, 300) nach einem der vorhergehenden Ansprüche, wobei
die mindestens eine Betriebsparametererfassungseinheit eine Energiespeicherzustandsermittlungseinheit umfasst, die ausgebildet ist, als einen Betriebsparameter Energiespeicherzustandsdaten (126) in Abhängigkeit eines Zustands der Energiespeichereinheit (120) bereitzustellen, und
die Lademanagementeinheit (140) ausgebildet ist, die Ladeobergrenze (LW1, LW2) und/oder den Alarmsignalschwellwert (AW1, AW2) und/oder die Ladespannung (142) und/oder den Ladestrom (142) in Abhängigkeit von den Energiespeicherzustandsdaten (126) festzulegen.

10. Steuereinrichtung (100, 300) nach einem der vorhergehenden Ansprüche, wobei
die mindestens eine Betriebsparametererfassungseinheit (380) eine Eingabeschnittstelle umfasst, die ausgebildet ist, den Betriebsparameter (382) anhand einer Benutzereingabe zu ermitteln, und
die Lademanagementeinheit (140) ausgebildet ist, die Ladeobergrenze (LW1, LW2) und/oder den Alarmsignalschwellwert (AW1, AW2) und/oder die Lade-spannung (142) und/oder den Ladestrom (142) in Abhängigkeit von der Benutzereingabe festzulegen.

11. Steuereinrichtung (100, 300) nach einem der vorhergehenden Ansprüche, wobei
die Lademanagementeinheit (140) ausgebildet ist, die elektrische Ladeleistung (144) an der Energiespeichereinheit (120) nur bereitzustellen, solange der Füllstand unterhalb der Ladeobergrenze (LW1, LW2) liegt, und/oder
die Lademanagementeinheit (140) ausgebildet ist, das Alarmsignal (346) bei einem Absinken des Füllstandes unterhalb des Alarmsignalschwellwertes (AW1, AW2) bereitzustellen.

12. Steuereinrichtung (100) nach einem der vorhergehenden Ansprüche, wobei die Lademanagementeinheit (140) ausgebildet ist, den Wert (142) der Ladespannung und/oder des Ladestroms durch Auswahl aus einer Vielzahl von vordefinierten Werteniveaus in Abhängigkeit des mindestens einen Betriebsparameters (172) festzulegen.

13. Steuereinrichtung (100) nach einem der vorhergehenden Ansprüche, wobei die Lademanagementeinheit (140) ausgebildet ist, den Wert der Ladespannung (142) und/oder des Ladestroms (142) so festzulegen, dass dieser in Abhängigkeit des mindestens einen Betriebsparameters (172) zeitabhängig zwischen zwei Extremwerten variiert.

14. Blutpumpeneinrichtung (500) umfassend:
eine Blutpumpe (190), und
eine Steuereinrichtung (100, 300) nach einem der vorhergehenden Ansprüche.

15. Verfahren (600) zum Betreiben einer Steuereinrichtung (100, 300) für eine Blutpumpe (190), umfassend die folgenden Schritte:
Bereitstellen (602) von elektrischer Energie mittels einer Energiespeichereinheit der Steuereinrichtung an einem Blutpumpenanschluss der Steuereinrichtung für einen Betrieb der Blutpumpe,
Empfangen (604) von elektrischer Energie über eine Ladeschnittstelle der Steuereinrichtung und Bereitstellen der elektrischen Energie in Form einer Ladeleistung an der Energiespeichereinheit zum Aufladen der Energiespeichereinheit,
Ermitteln (606) mindestens eines Betriebsparameters der Steuer-einrichtung,
Festlegen (608) eines Wertes einer Ladespannung und/oder eines Wertes eines Ladestroms für die an der Energiespeichereinheit bereitgestellte Ladeleistung unter Benutzung eines Füllstandes der Energiespeichereinheit und mindestens einem der Betriebsparameter, und/oder
Festlegen (608) einer Ladeobergrenze, bis zu der die elektrische Energiespeichereinheit aufgeladen wird, unter Benutzung von mindestens einem der Betriebsparameter, und/oder
Festlegen (608) eines Alarmsignalschwellwertes, in Abhängigkeit dessen ein Alarmsignal bereitgestellt wird, unter Benutzung von mindestens einem der Betriebsparameter.
